# EUROPEAN PATENT SPECIFICATION

(11) **EP 3 284 422 B1**
(45) Date of publication and mention of the grant of the patent: **24.02.2021**
(21) Application number: 15889168.9
(22) Date of filing: 15.04.2015
(51) Int. Cl.: A61B 17/29, A61B 18/14

(54) **HINGE MEMBER FOR BENDABLE TREATMENT TOOL AND BENDABLE TREATMENT TOOL HAVING SAID HINGE MEMBER INCORPORATED THEREIN**
SCHARNIERELEMENT FÜR BIEGBARES BEHANDLUNGSWERKZEUG UND BIEGBARES BEHANDLUNGSWERKZEUG MIT SOLCH EINEM SCHARNIER
ÉLÉMENT D'ARTICULATION POUR OUTIL DE TRAITEMENT PLIABLE ET OUTIL DE TRAITEMENT PLIABLE AYANT LEDIT ÉLÉMENT D'ARTICULATION INCORPORÉ EN SON SEIN

(43) Date of publication of application: 21.02.2018
(73) Proprietor: Kyushu University National University Corporation, Fukuoka-shi Fukuoka 812-8581 (JP); Hogy Medical Co., Ltd., Tokyo 107-8615 (JP)
(72) Inventor: NAKADATE, Ryu, Fukuoka-shi, Fukuoka 812-8581 (JP); KENMOTSU, Hajime, Fukuoka-shi, Fukuoka 812-8581 (JP); HASHIZUME, Makoto, Fukuoka-shi, Fukuoka 812-8581 (JP); FUJITA, Hiroyasu, Tokyo 107-8615 (JP); NAGAI, Shunsuke, Tokyo 107-8615 (JP); KATO, Jiro, Tokyo 107-8615 (JP)
(74) Representative: Viering, Jentschura & Partner mbB Patent- und Rechtsanwälte
(86) International application number: PCT/JP2015/061566
(87) International publication number: WO 2016/166830

(56) References cited:
- WO-A1-2008/108030
- WO-A2-2004/086957
- JP-A- 2006 521 882
- JP-A- 2009 101 134
- US-A1- 2007 038 230
- US-A1- 2009 054 734
- US-A1- 2010 010 309
- US-A1- 2014 135 685
- US-A1- 2014 275 799
- US-B1- 6 743 239

## Description

### Technical Field

The present invention relates to a bendable, bending treatment instrument inserted into a flexible endoscope, and specifically, to a bending treatment instrument hinge member and a bending treatment instrument incorporating the hinge member, where the bending treatment instrument hinge member and bending treatment instrument are inserted into a treatment instrument channel of a flexible endoscope or a treatment instrument passage tube attached to a flexible endoscope, are caused to reach an abdominal organ such as the stomach or intestines together with the flexible endoscope from the mouth or anus, and are used to allow a distal end of a treatment instrument such as a scalpel or forceps for resecting cancer such as epithelial cancer to freely bend independently of the flexible endoscope.

### Background Art

In recent years, operative procedures such as endoscopic submucosal dissection (ESD) have come to be used, where ESD involves inserting a treatment instrument through the mouth or anus and removing one slice from an upper layer of a mucous membrane over a wide area of the stomach or large intestine without relying on a laparotomy or arthroscopic surgery. Furthermore, an operative procedure (NOTES: Natural Orifice Translumenal Endoscopic Surgery) is known which involves inserting a flexible endoscope such as an upper or lower gastrointestinal endoscope through the mouth, anus, vagina, or urethra which originally exists in the surface of the body, then taking the flexible endoscope to an abdominal cavity by penetrating a stomach or large-intestine wall, and conducting diagnosis or treatment on an abdominal organ.

Since the natural orifice translumenal endoscopic surgery typified by endoscopic submucosal dissection (ESD) conducts treatment or the like by inserting a treatment instrument such as forceps or a scalpel together with a flexible endoscope through the mouth or the like which originally exists in the surface of the body, and taking the treatment instrument to an affected part, the surgery causes no damage to the surface of the body, can reduce the risk of complications such as infection or adhesion of the abdominal wall, which accompany ordinary surgery, and can reduce stress on the human body.

As described in Patent Literature 1, the treatment instrument used for the natural orifice translumenal endoscopic surgery includes a bending portion inserted into the flexible endoscope and used to bendably manipulate the treatment instrument projecting from a distal end of the flexible endoscope. Also, the treatment instrument includes a sheath and wire unit adapted to transmit bending motion to the bending portion and an operating portion used to manipulate the bending motion of the bending portion by pushing and pulling the sheath and wire.

Also, regarding a configuration of the bending portion, as described in Patent Literature 2, a structure of a flexible endoscope is known in which a bending portion is constructed by combining plural joint rings, which are provided with concavity and convexity in an axial direction as well as with a through-hole to allow passage of wires.

This configuration makes it possible to use transmission motion of the wires reliably as bending motion and withstand a load resulting from resection motion or grasping motion of forceps or scalpel attached to a distal end. Patent literature 3 describes a hinge member having the features of the preamble.

### Citation List

### Patent Literature

Patent Literature 1: Japanese Patent Laid-Open No. 2010-511440
Patent Literature 2: Japanese Patent Laid-Open No. 2005-7068
Patent Literature 3: WO 2004/086957 A2.

### Summary of Invention

### Technical Problem

However, the bending treatment instrument described in Patent Literature 1 has an outside diameter of approximately 4.0 mm, which is a size not suitable for passage through an endoscope channel of the endoscope. Thus, the bending treatment instrument cannot be taken safely to the stomach or the like from the mouth through the esophagus and is practically unusable. On the other hand, any attempt to reduce the outside diameter to such a size as to enable insertion into the endoscope channel to allow application to the above-mentioned endoscopic submucosal dissection (ESD) will obstruct bendability of the treatment instrument as well as appropriate grasping with forceps and resection with a scalpel, and consequently appropriate reduction of the diameter has not yet been achieved.

Furthermore, with the treatment instrument which is described in Patent Literature 1, because a wire is passed through a control wire lumen formed in a mesh layer, there is a problem in that it is difficult to withstand the loads resulting from the grasping motion of the forceps and the resection motion of the scalpel, making resection and ablation operations very difficult. Also, with the configuration which is described in Patent Literature 2, although various shapes are known concerning the endoscope channel of the flexible endoscope, including those with diameter sizes of about 3.8 mm, 3.2 mm, and 2.8 mm, any of the shapes is small in diameter, which poses a problem in that it is difficult for the bending portion to be inserted into the endoscope channel and used as a bendable treatment instrument, because of increased loads applied to the bending portion.

The present invention has been made to solve the above problems and specifically has an object to provide a hinge member of a bending treatment instrument inserted into an endoscope channel and used bendably through intuition and capable of withstanding loads resulting from grasping and resection motions of treatment instruments as well as to provide a bending treatment instrument which uses the hinge member.

### Solution to Problem

To solve the above problem, the present invention provides a hinge member substantially tubular in shape, where a plurality of the hinge members make up a bending treatment instrument by being arranged in a row substantially coaxially with one another, claimed in claim 1. Further embodiments of the invention are defined by claims 2-11.

The hinge member according to the present invention further comprises a device-wire passage hole penetrating an axial center portion.

Also, in the hinge member according to the present invention, preferably the device-wire passage hole is formed into a substantially elliptical shape in a cross section orthogonal to the axial direction.

Also, in the hinge member according to the present invention, preferably the substantially elliptical shape of the device-wire passage hole is formed such that a major axis is parallel to a direction orthogonal to an opposing direction of the projections.

In the hinge member according to the present invention, the bending-wire passage hole is formed at opposite ends of a pair of the projections for a total of four.

In the hinge member according to the present invention, each of the projections protrudes by forming a substantially arc shape and the recess is formed into an arc shape corresponding to the projections so as to be able to come into sliding contact with the projections.

Also, preferably the hinge member according to the present invention further comprises a device-wire passage hole penetrating an axial center portion, wherein the bending-wire passage hole increases in diameter toward the end faces on the distal side and the proximal side from a center portion of the bending-wire passage hole along the axial direction.

Also, in the hinge member according to the present invention, preferably the recess includes a curved portion and a straight portion extending from an end portion of the curved portion.

Also, in the hinge member according to the present invention, preferably a curvature of the curved portion is substantially equal to a curvature of tips of the projections.

Also, in the hinge member according to the present invention, preferably notches facing each other are formed, respectively, at those positions on both flanks of each of the projections which are continuous with the end face on the distal side.

Also, in the hinge member according to the present invention, preferably the recess and the projections are each formed into an arc shape; and a curvature of the recess is larger than a curvature of the projections.

Also, in the hinge member according to the present invention, preferably a diameter size in the cross section orthogonal to the axial direction of the hinge member is 3.8 mm or below.

Also, the present invention provides a bending treatment instrument formed by assembling the plurality of hinge members.

Also, in the bending treatment instrument according to the present invention, preferably the bending treatment instrument is inserted into an endoscope channel.

### Advantageous Effects of Invention

According to the present invention, since the bending-wire passage hole is formed at a position avoiding tips of the projections, a bending wire passed through the bending-wire passage hole bends more gently together with the bending treatment instrument, making it possible to perform push/pull motion of the bending wire with a smaller force.

Also, according to the present invention, since the device-wire passage hole is formed in a substantially elliptical shape in a cross section orthogonal to the axial direction, it is possible to expand a range of bending motion of a device wire passed through the device-wire passage hole.

Also, according to the present invention, since the device-wire passage hole is formed such that a major axis is parallel to a direction orthogonal to an opposing direction of the projections, even if an outside diameter size of the hinge member is minimized, thickness of the projections can be secured, improving load-bearing capacity during the bending motion.

Also, according to the present invention, since the bending-wire passage hole is formed at opposite ends of a pair of the projections for a total of four, it is possible to perform bending motion with two degrees of freedom.

Also, according to the present invention, since each of the projections protrudes by forming a substantially arc shape and the recess and the projections are formed in arc shapes so as to be able to come into sliding contact with each other, it is possible to implement smooth bending motion.

Also, according to the present invention, since the bending-wire passage hole is formed in a so-called tapered shape, increasing in diameter toward the end faces on the distal side and the proximal side from a center portion of the bending-wire passage hole along the axial direction, a bending wire passed through the bending-wire passage hole bends more gently together with the bending treatment instrument, making it possible to perform push/pull motion of the bending wire with a smaller force.

Also, according to the present invention, since the recess includes a curved portion and a straight portion extending from an end portion of the curved portion, adjacent hinge members are shaped to fit inside one another during bending, allowing the bending to be done to the limit without causing much strain.

Also, according to the present invention, since a curvature of the curved portion is formed so as to be substantially equal to a curvature of tips of the projections, adjacent hinge members are shaped to fit inside one another during bending, allowing the bending to be done to the limit without causing much strain.

Also, according to the present invention, since notches facing each other are formed, respectively, at those positions on both flanks of each of the projections which are continuous with the end face on the distal side, when the bending treatment instrument bends, an end portion of the hinge member on the proximal side slides along an adjacent projection, allowing an inclination angle on the proximal side to be increased and thereby making it possible to increase a bending angle of the bending treatment instrument.

Also, according to the present invention, since the recess and the projections are each formed in an arc shape and a curvature of the recess is larger than a curvature of the projections, when the bending treatment instrument bends, the recess and the projections can slide on each other smoothly.

Also, according to the present invention, since a diameter size in the cross section orthogonal to the axial direction of the hinge member is 3.8 mm or below, it is possible to configure a bending treatment instrument which can be used by being inserted into an endoscope channel.

### Brief Description of Drawings

[FIG. 1] FIG. 1 is a schematic diagram illustrating how a bending treatment instrument according to the present embodiment is used.
[FIG. 2] FIG. 2 is a schematic diagram illustrating an example of use of the bending treatment instrument according to the present embodiment.
[FIG. 3] FIG. 3 is a side view illustrating a configuration of a forceps-equipped bending treatment instrument according to the present embodiment.
[FIG. 4] FIG. 4 is an enlarged view illustrating a configuration of a bending portion of the forceps-equipped bending treatment instrument.
[FIG. 5] FIG. 5 is a longitudinal sectional view of FIG. 4.
[FIG. 6] FIG. 6 is a configuration diagram of a hinge top located on a proximal side of the bending portion.
[FIG. 7] FIGS. 7(a) and 7(b) are partial sectional views illustrating open/close motion of forceps, where FIG. 7(a) shows a closed state and FIG. 7(b) shows an open state.
[FIG. 8] FIG. 8 is an enlarged view illustrating a configuration of a bending portion of a scalpel-equipped bending treatment instrument according to the present embodiment.
[FIG. 9] FIG. 9 is a perspective view of hinge members making up the bending portion.
[FIG. 10] FIG. 10 is a side view of the hinge member.
[FIG. 11] FIG. 11 is a top view of the hinge member.
[FIG. 12] FIG. 12 is a front view of the hinge member.
[FIG. 13] FIGS. 13(a) to 13(c) are configuration diagrams illustrating a configuration of a sheath and wire unit, where FIG. 13(a) is a configuration diagram illustrating a configuration of the sheath and wire unit, and FIG. 13(b) is a diagram illustrating a variation of a bending wire and device wire, and FIG. 13(c) is a sectional view of the variation of the bending wire and device wire.
[FIG. 14] FIG. 14 is a sectional view of the sheath and wire unit.
[FIG. 15] FIG. 15 is a perspective view of a variation of the hinge member making up the bending portion.
[FIG. 16] FIGS. 16(a) and 16(b) show the variation of the hinge member according to the present embodiment, where FIG. 16(a) is a sectional view taken along line A-A in FIG. 15 and FIG. 16(b) is a side view of a second variation of the hinge member according to the present embodiment.
[FIG. 17] FIG. 17 is a side view of the variation of the hinge member.
[FIG. 18] FIG. 18 is a sectional view illustrating bending motion of the bending portion.

### Description of Embodiment

A hinge member according to the present invention and a bending treatment instrument constructed by assembling the hinge members will be described below with reference to the drawings. Note that the embodiment described below is not intended to limit the claimed invention and that a combination of all the features described in the embodiment are not necessarily essential for the means to solve the problem according to the present invention.

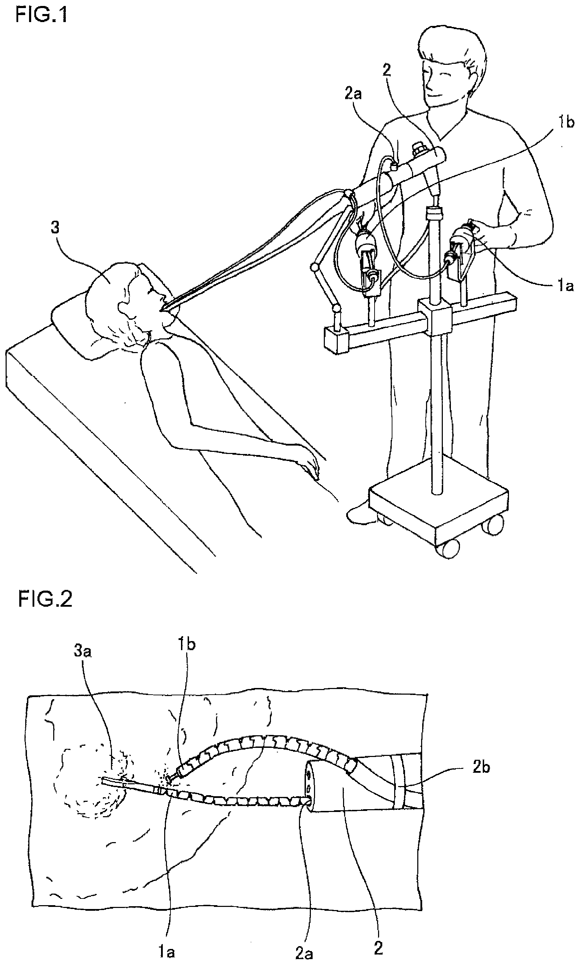

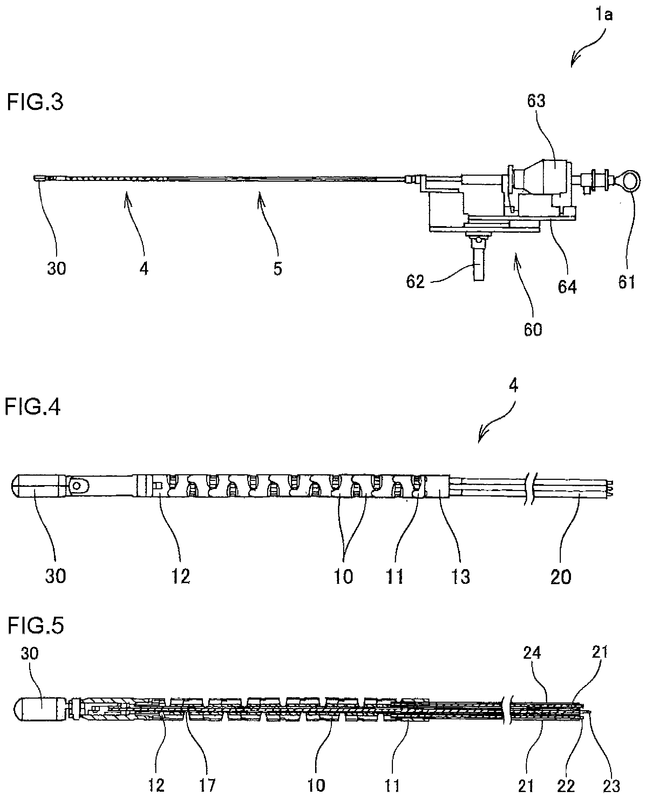

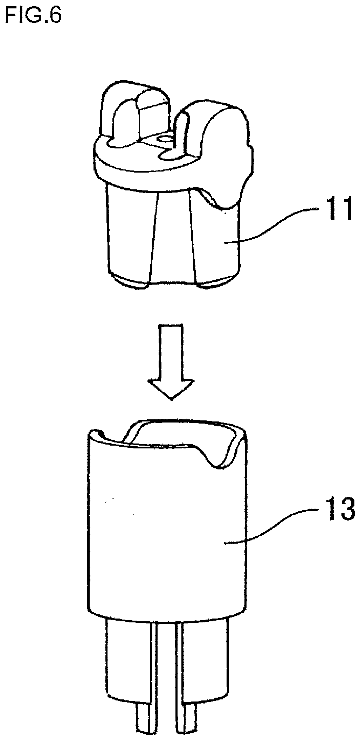

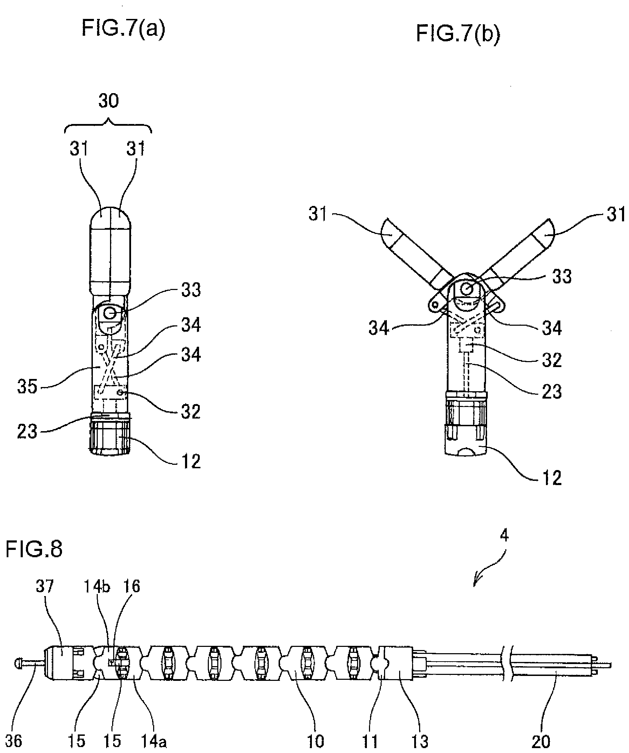

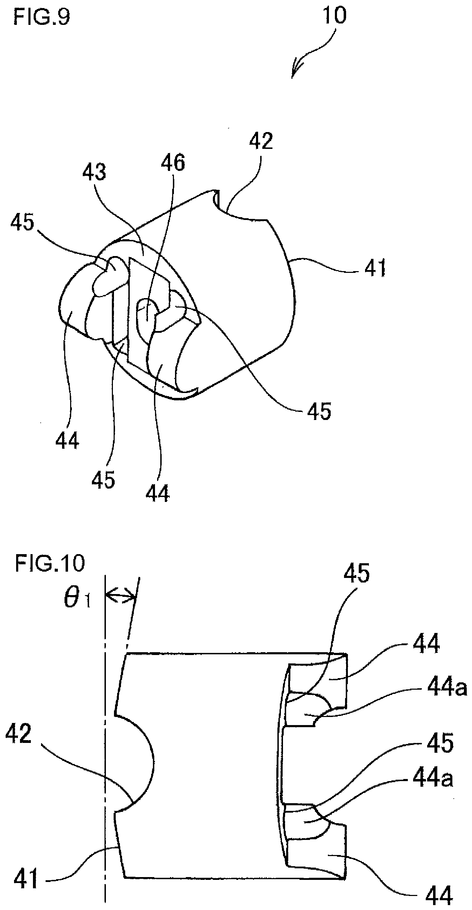

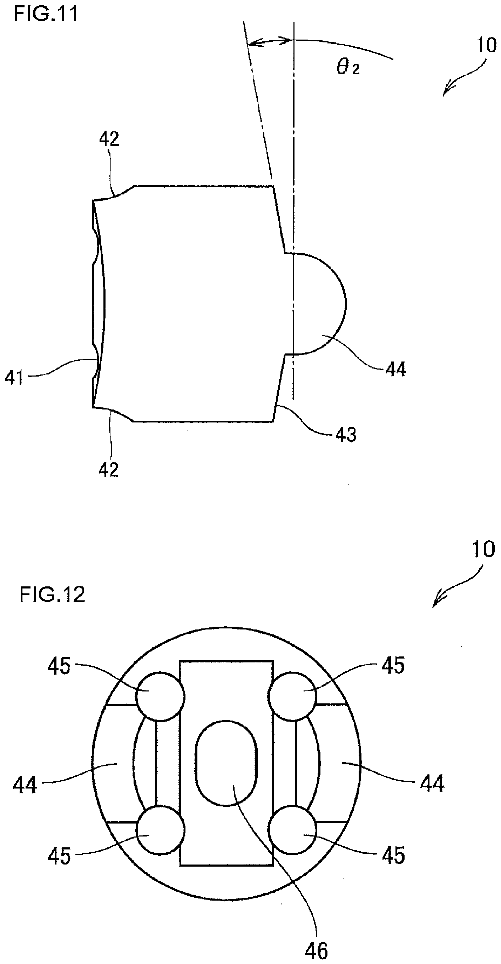

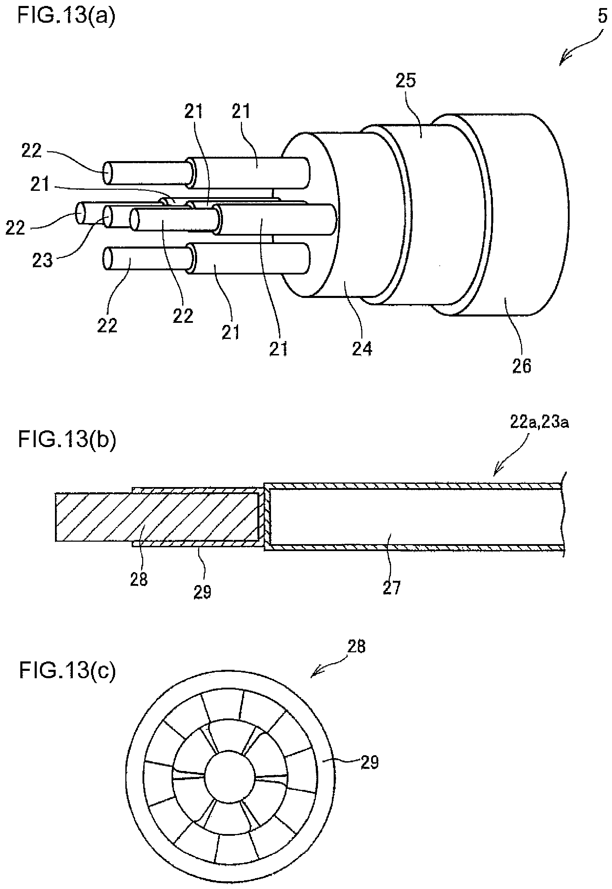

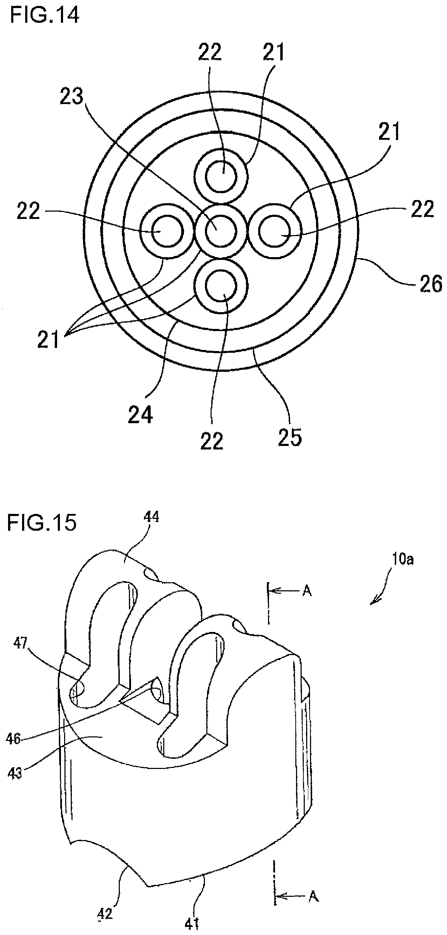

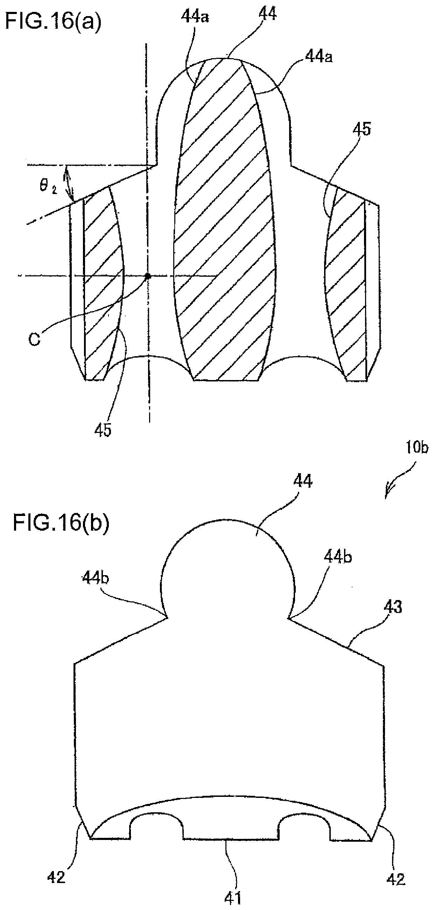

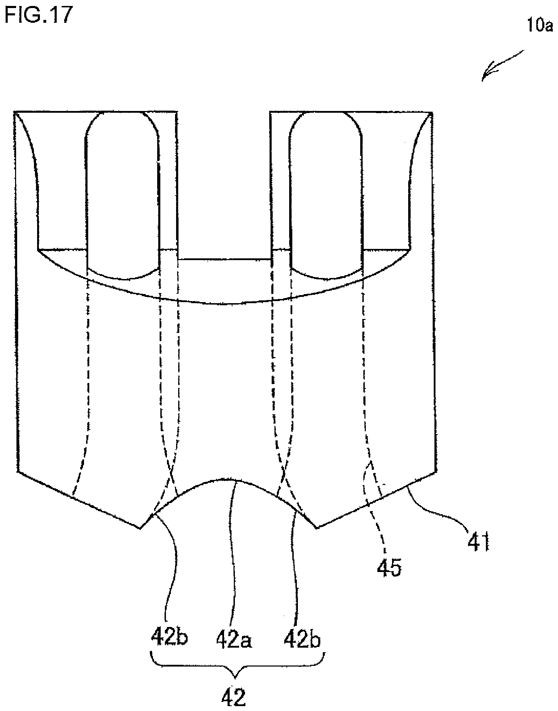

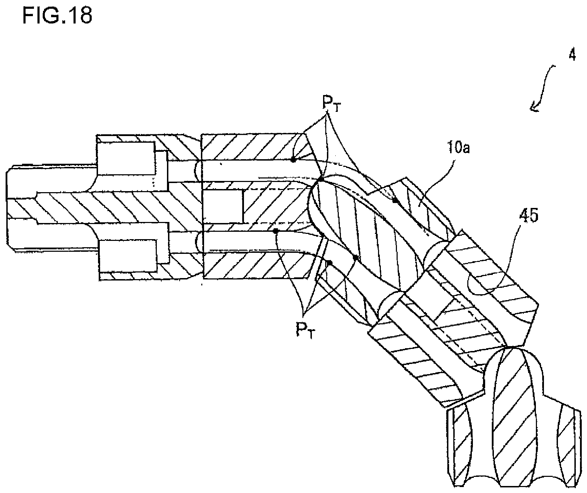

FIG. 1 is a schematic diagram illustrating how a bending treatment instrument according to the present embodiment is used; FIG. 2 is a schematic diagram illustrating an example of use of the bending treatment instrument according to the present embodiment; FIG. 3 is a side view illustrating a configuration of a forceps-equipped bending treatment instrument according to the present embodiment; FIG. 4 is an enlarged view illustrating a configuration of a bending portion of the forceps-equipped bending treatment instrument; FIG. 5 is a longitudinal sectional view of FIG. 4; FIG. 6 is a configuration diagram of a hinge top located on a proximal side of the bending portion; FIGS. 7(a) and 7(b) are partial sectional views illustrating open/close motion of forceps, where FIG. 7(a) shows a closed state and FIG. 7(b) shows an open state; FIG. 8 is an enlarged view illustrating a configuration of a bending portion of a scalpel-equipped bending treatment instrument according to the present embodiment; FIG. 9 is a perspective view of hinge members making up the bending portion; FIG. 10 is a side view of the hinge member; FIG. 11 is a top view of the hinge member; FIG. 12 is a front view of the hinge member; FIGS. 13(a) to 13(c) are configuration diagrams illustrating a configuration of a sheath and wire unit, where FIG. 13(a) is a configuration diagram illustrating a configuration of the sheath and wire unit, and FIG. 13(b) is a diagram illustrating a variation of a bending wire and device wire, and FIG. 13(c) is a sectional view of the variation of the bending wire and device wire; FIG. 14 is a sectional view of the sheath and wire unit; FIG. 15 is a perspective view of a variation of the hinge member making up the bending portion; FIGS. 16(a) and 16(b) show the variation of the hinge member according to the present embodiment, where FIG. 16(a) is a sectional view taken along line A-A in FIG. 15 and FIG. 16(b) is a side view of a second variation of the hinge member according to the present embodiment; FIG. 17 is a side view of the variation of the hinge member; and FIG. 18 is a sectional view illustrating bending motion of the bending portion.

As shown in FIGS. 1 and 2, the bending treatment instrument according to the present embodiment includes a forceps-equipped bending treatment instrument 1a equipped with forceps at a distal end and a scalpel-equipped bending treatment instrument 1b equipped with an electric scalpel at a distal end. The bending treatment instruments 1a and 1b are inserted into an endoscope channel 2a of a flexible endoscope 2 or into a treatment instrument passage tube 2b attached to a distal end of the flexible endoscope 2, are inserted together with the flexible endoscope 2 through the mouth or anus of a patient 3, and are used to diagnose or resect an affected part 3a such as cancer in the digestive tract or the like.

In so doing, the forceps-equipped bending treatment instrument 1a and scalpel-equipped bending treatment instrument 1b bend in a separate manner independently of the flexible endoscope 2 to provide at least two degrees of freedom, making it possible to grasp or resect the affected part 3a with a point of view of the flexible endoscope 2 fixed and carry out a procedure with a stable field of view and a high degree of freedom.

As shown in FIG. 3, the forceps-equipped bending treatment instrument 1a includes forceps 30 attached to a distal end of a bending portion 4 having two degrees of freedom in horizontal and vertical directions, an operating portion 60 used to perform bending motion of the bending portion 4 and open/close motion of the forceps 30, and a sheath and wire unit 5 equipped with plural wires adapted to transmit manipulation of the operating portion 60 and sheaths through which the wires are passed.

By making a grip 61 pivot up, down, left, and right like a joystick relative to the operating portion body 63, with the grip 61 being connected to plural wires passed through the sheath and wire unit 5, the operating portion 60 pushes and pulls the wires passed through the bending portion 4 and connected thereto in the longitudinal direction and thereby causes the bending portion 4 to perform bending motion. Besides, the grip 61 can also be used to perform push/pull motion in the longitudinal direction and thereby push and pull the wire connected to the forceps 30, effecting open/close motion of the forceps 30.

Furthermore, the operating portion 60 is attached to a base 62 via a slider mechanism 64 configured to be able to slide an operating portion body 63 in a longitudinal direction. By sliding the slider mechanism 64 along the longitudinal direction, the operating portion 60 allows the forceps 30, the bending portion 4, and the sheath and wire unit 5 to be pushed and pulled along the longitudinal direction and makes it possible to adjust an amount of protrusion of the forceps 30 from the endoscope channel 2a or treatment instrument passage tube 2b.

As shown in FIG. 4, the bending portion 4 has the forceps 30 attached on a distal side via a hinge end 12, and has a sheath 20 of the sheath and wire unit 5 attached on a proximal side via a hinge top 11 and hinge base 13. Also, the bending portion 4 includes plural hinge members 10 arranged in a row coaxially with one another, and performs bending motion as adjacent hinge members 10 slide on one another in a direction orthogonal to an axial direction.

Specifically, as shown in FIG. 5, the bending portion 4 has plural bending wires 22 passed therethrough, with the bending wires 22 being connected at one end to the operating portion 60, and at another end to the hinge end 12, and effects bending motion by allowing the bending wires 22 to be pushed and pulled via the operating portion 60 and thereby making the hinge members 10 slide on one another. Note that the bending wires 22 and hinge end 12 may be fixed to each other by bonding or by attaching a caulking member to each of the bending wires 22 and hooking the caulking member onto the hinge end 12 through swaging. If the bending wires 22 and hinge end 12 are fixed to each other using caulking members in this way, strength of a connecting portion between the bending wires 22 and hinge end 12 can be improved using a simple configuration, and even if stresses caused by pushing and pulling the bending wires 22 to bend the bending portion 4 are concentrated on the connecting portion, peeling of the bending wires 22 can be prevented. Note that there is no particular limit to the material and shape of the caulking member as long as the strength of the connecting portion can be improved as described above, but specifically a columnar member made of metal can be suitably used.

Also, being connected at one end to the operating portion 60, and at another end to the forceps 30, a device wire 23 is passed through the hinge members 10 by penetrating an axial center portion of the hinge members 10, and is pushed and pulled to effect open/close motion of the forceps 30.
Furthermore, the device wire 23 is passed through a fluorocarbon resin tube 17 to reduce sliding resistance with the hinge members 10 when the device wire 23 is pushed and pulled inside the bending portion 4 and prevent the hinge members 10 from being displaced from one another in a radial direction.

The bending wires 22 and device wire 23 are passed, respectively, through under-mentioned inner sheaths 21 attached to the hinge top 11. Each of the bending wires 22 is a stranded wire formed by twisting together plural stainless steel strands while the device wire 23 is a stranded wire formed by twisting together a smaller number of thicker stainless steel strands than the bending wire 22, and the bending wire 22 and device wire 23 have substantially the same outside diameter size. Specifically, the bending wire 22 is preferably a stranded wire formed by twisting together nineteen stainless steel strands and preferably a stranded wire formed by twisting together seven stainless steel strands is used as the device wire 23. This configuration gives the bending wires 22 enough resilience to bend easily while giving the device wire 23 appropriate rigidity needed to open and close the forceps 30 and push and pull the electric scalpel 36.

Also, the bending wires 22 and device wire 23 have been surface-treated to reduce sliding resistance in the inner sheaths 21. Note that preferably fluorocarbon resin, such as polytetrafluoroethylene (PTFE), or fluorinated carbon resin is used for the surface treatment.

Also, the sheath 20 extends to the hinge top 11, which is fitted into the hinge base 13 as shown in FIG. 6. Consequently, of the inner sheaths 21 making up the sheath 20, the inner sheaths 21 through which the bending wires 22 are passed are guided outward. This configuration allows the bending wires 22 to be guided smoothly from the sheath 20 to the bending portion 4. Note that the inner sheaths 21 are joined to an end face of the hinge top 11 by bonding or welding.

As shown in FIG. 7, the forceps 30 perform open/close motion as a pair of forceps blades 31 pivot relative to each other with a pin 33 serving as a pivot axis. The forceps blades 31 are attached on the proximal side and connected with open/close wires 34 intersecting each other, and the open/close wires 34 are connected to a mobile body 32 configured to move along with push/pull motion of the device wire 23. Note that the mobile body 32 and open/close wires 34 are housed in a forceps base 35.

With this configuration, when the device wire 23 is pulled, as shown in FIG. 7(a), the mobile body 32 moves toward the hinge end 12, making the open/close wires 34 intersect each other at such an intersection angle that will be acute with respect to the axial direction. In so doing, ends of the open/close wires 34 connected to the forceps blades 31 come close to each other, and consequently the forceps blades 31 are closed along with movement of the open/close wires 34.

Also, when the device wire 23 is pushed, as shown in FIG. 7(b), the mobile body 32 is pushed toward the forceps blades 31 by being spaced away from the hinge end 12. In so doing, the open/close wires 34 intersect each other at such an intersection angle that will be obtuse with respect to the axial direction, and consequently the ends of the open/close wires 34 connected to the forceps blades 31 are spaced away from each other, causing the forceps blades 31 to open along with the movement of the open/close wires 34.

In contrast, as shown in FIG. 8, the electric scalpel 36 is attached to the scalpel-equipped bending treatment instrument 1b via a distal portion 37. Configurations of the bending portion 4 and sheath 20 are similar to those of the forceps-equipped bending treatment instrument 1a described above, and thus detailed description thereof will be omitted.

The electric scalpel 36 is constructed from an electrically conductive material and adapted to resect or cauterize an affected part by conducting a high-frequency current, and the distal end is formed, for example, into a spherical shape or hook shape. Also, an amount of protrusion of the distal portion 37 is configured to be adjustable appropriately by pushing and pulling the device wire 23.

Also, the scalpel-equipped bending treatment instrument 1b has displacement prevention members 14a and 14b interposed between the distal portion 37 and hinge members 10. On the displacement prevention members 14a and 14b, as with the hinge members 10, projections protruding in the axial direction and a recess are formed on the end faces in the axial direction, a claw 15 protruding in the axial direction is formed at one end in the axial direction, and a groove 16 engaged with the claw 15 is formed at another end. As the claw 15 and groove 16 are engaged with each other, the displacement prevention members 14a and 14b prevent the distal portion 37 from being displaced relative to the bending portion 4 in the radial direction.

As shown in FIG. 9, the hinge members 10 are substantially cylindrical members of 3.8 mm or below in outside diameter. Preferably the outside diameters of the hinge members 10 and the sheath and wire unit 5 are 3.8 mm, more preferably 3.2 mm, and most preferably 2.8 mm. These sizes allow the bending treatment instrument 1 to be inserted into the endoscope channel 2a. Also, each of the hinge members 10 has a recess 42 formed along the radial direction on an outer edge of a proximal end face 41 on the proximal side and has a pair of projections 44 formed on a distal end face 43 on the distal side, protruding in the axial direction and placed facing each other along the radial direction.

Also, the recess 42 and projections 44 are placed being shifted 90 degrees in a circumferential direction from each other. Furthermore, the projections 44 are each formed in an arc shape so as to be slidable with respect to the recess 42 of the adjacent hinge member 10. Note that the recess 42 is also formed in an arc shape of substantially the same curvature as the arc shape of the projections 44 by corresponding thereto.

Each of the hinge members 10 has plural bending-wire passage holes 45 formed penetrating therethrough in parallel to the axial direction and has a device-wire passage hole 46 formed penetrating the axial center portion. Four bending-wire passage holes 45 are arranged substantially at equal intervals in the circumferential direction at positions on the distal end face 43 avoiding sliding surfaces of the projections 44 with respect to the recess 42. In this way, since the bending-wire passage holes 45 are formed at positions avoiding the sliding surfaces of the projections 44 with respect to the recess 42, when the adjacent projections 44 and recess 42 slide on each other, bending the bending portion 4, the bending wires 22 passed through the bending-wire passage holes 45 do not interfere the sliding between the projections 44 and recess 42 and smooth bending motion can be implemented. Note that the bending-wire passage hole 45 is formed at opposite ends of each of the two projections 44 for a total of four locations.

Also, as shown in FIG. 10, in each of the projections 44, an interference prevention groove 44a adapted to prevent interference with the bending wires 22 is formed by continuing from an outer edge of the bending-wire passage hole 45. Furthermore, the proximal end face 41 is formed as an inclined surface inclined at a predetermined angle θ1, the distal end face 43 is also formed as an inclined surface inclined at a predetermined angle θ2 as shown in FIG. 11, and the inclined surfaces prevent the adjacent distal end face 43 and proximal end face 41 from interfering with each other when the bending portion 4 bends.

As shown in FIG. 12, the device-wire passage hole 46 is formed in an elliptical shape. As the device-wire passage hole 46 is formed in an elliptical shape, a motion range of the device wire 23 passed through the device-wire passage hole 46 with the bending portion 4 being in a bent state can be secured in a major axis direction. This enables smooth push/pull motion of the device wire 23 even if the bending portion 4 is in a bent state.

Furthermore, a major axis of the device-wire passage hole 46 is parallel to a direction orthogonal to an opposing direction of the projections 44. This configuration allows wall thickness of the projections 44 to be increased, making it possible to secure large sliding surfaces on the projections 44 and ensure rigidity of the bending portion 4.

As shown in FIGS. 13 and 14, the sheath and wire unit 5 includes plural inner sheaths 21 through which plural bending wires 22 and a device wire 23 are passed, respectively, an outer sheath 24 through which the inner sheaths 21 are passed all together, a liner blade 25 covering an outer surface of the outer sheath 24, and a protective tube 26 covering an outer surface of the liner blade 25.

The inner sheaths 21, which guide push/pull motion of the bending wires 22 and device wire 23 and prevent the wires from interfering with each other, are so-called close-wound coils each formed by closely winding a flat metal wire with a flat cross section into a spiral. The use of flat wires makes it possible to ensure strength of the inner sheaths 21 and increase an inside diameter size, and allows the bending wires 22 and device wire 23 passed through the inner sheaths 21 to perform push/pull motion smoothly in the inner sheaths 21. Also, if width of the flat wires used is increased relative to a diameter of the inner sheaths 21, it is possible to increase the strength and reduce frictional resistance resulting from the push/pull motion of the bending wires 22 and device wire 23 in the inner sheaths 21. Specifically, if X denotes the thickness of the flat wires for the inner sheaths 21 and Y denotes the width of the flat wires, preferably X:Y = 1:10. Furthermore, as the wires are configured into close-wound coils, the inner sheaths 21 are configured not to be buckled or shrunk and stretched by the push/pull motion of the bending wires 22 and device wire 23.

In contrast, if a round wire with a circular cross section is used for each of the inner sheaths 21, because adjoining turns of the round wire come into line contact with each other when the wire is wound into a spiral, if the inner sheaths 21 are bent when the bending treatment instruments are inserted into the endoscope channel or stored, position of the line contact moves in a circumferential direction, which poses a problem in that the round wires are buckled, causing shrinkage of the inner sheaths 21. Also, if round wires are close-wound, there is not only the problem of buckling resulting in shrinkage, but also a problem of irreversible deformation. Also, regarding the inner sheaths 21, as shown in FIG. 14, the inner sheaths 21 through which the bending wires 22 are passed are arranged in the circumferential direction around the inner sheath 21 through which the device wire 23 is passed.

The outer sheath 24, which is a member making up a framework of the sheath and wire unit 5, protects the bending wires 22 and device wire 23 and transmits a turning force of the entire bending treatment instruments. As with the inner sheaths 21, the outer sheath 24 is formed by winding a flat metal wire with a flat cross section into a spiral, but is configured as a so-called coarse-wound coil having predetermined gaps. As the wire is configured into a coarse-wound coil in this way, resilience in a bending direction is improved and even if bent to a small radius, the sheath and wire unit 5 is not buckled and can bend smoothly by following the bending of the endoscope channel. Note that since the inner sheaths 21 themselves are prevented from shrinking by being wound closely as described above, even if the outer sheath 24 is wound coarsely, shrinkage of the sheath and wire unit 5 can be inhibited as much as possible.

When an external force acts on the bending portion 4 due to a load exerted by the grasping motion of the forceps 30, resection motion of the electric scalpel 36 or the like, the liner blade 25 prevents runout of an operating axis caused by the external force, and a mesh structure formed by cross-weaving metal wire strands is preferably used.

The protective tube 26 is a member adapted to cover and protect the sheath and wire unit 5 and electrically insulate a high-frequency high-voltage source applied to the electric scalpel 36. Specifically, it is suitable to use a heat-shrinkable tube made of polyolefin or the like.

Furthermore, various changes can be made to the hinge member to increase bending of the bending portion 4. For example, FIG. 15 shows a variation of the hinge member used for the bending treatment instrument according to the present embodiment. As shown in FIG. 17, in the hinge member 10a according to the variation, the recess 42 includes a curved portion 42a having a curvature substantially equal to a curvature of the projections 44, and straight portions 42b extending from opposite ends of the curved portion 42a. As the recess 42 and projections 44 are formed in this way, the adjacent hinge members 10a are shaped to fit inside one another during bending, allowing the bending to be done to the limit without causing much strain.

Also, as shown in FIG. 16(a), in each of the projections 44, the interference prevention groove 44a adapted to prevent interference with the bending wires 22 is formed by continuing from an outer edge of the bending-wire passage hole 45. Furthermore, the bending-wire passage hole 45 is formed in a so-called tapered shape, increasing in diameter toward the end face 43 on the distal side and the end face 41 on the proximal side from a center portion c along the axial direction. Note that the proximal end face 41 is formed as an inclined surface inclined at a predetermined angle, that the distal end face 43 is also formed as an inclined surface inclined at a predetermined angle θ2, and that the inclined surfaces prevent the adjacent distal end face 43 and proximal end face 41 from interfering with each other when the bending portion 4 bends.

With the hinge members 10a being configured in this way, as shown in FIG. 18, the bending wires passed through the bending-wire passage holes 45 stick to the bending-wire passage holes 45 on an outer side in a bending direction by being pulled along with bending of the bending treatment instrument, and thereby come into contact with contact points Pt on an inner circumferential surface of the bending-wire passage holes 45. The bending-wire passage holes 45 in the hinge member 10a according to the present embodiment are each formed in a tapered shape, increasing in diameter toward the opposite end faces as described above. This makes it possible to increase a space in which the bending wires are passed and which is defined by the adjacent bending-wire passage holes 45 during bending and thereby increase the bending angle. Also, a motion space of the bending wires is increased as well, improving operability of the bending treatment instrument operated by pushing and pulling the wires.

Also, as shown in FIG. 16(b), notches 44b cut facing each other may be formed at those positions on both lateral flanks of each projection 44 which are continuous with the end face 43 on the distal side. By forming the notches 44b in this way, it is possible to carry out a design in such a way as to maximize a length of an outer circumferential surface of the projection 44. Besides, the notches 44b make it possible to reduce a region of interference between adjoining hinge members 10b when the bending treatment instrument bends, increase the inclination angles of the hinge members, and thereby increase the bending angle of the bending treatment instrument. In so doing, the recess 42 is formed in an arc shape by corresponding to the arc shape of the projections 44 and preferably a curvature of the recess 42 is larger than a curvature of the projections 44.

In this way, with the bending treatment instrument according to the present embodiment, even if outside diameters of the bending portion 4 and the sheath and wire unit 5 are minimized to 3.8 mm or below to allow the bending portion 4 and the sheath and wire unit 5 to be passed through the endoscope channel, when the bending portion 4 performs bending motion, the bending motion is not obstructed by interference of the hinge members 10 with the bending wires 22 or device wire 23 passed through the bending portion 4, and the push/pull motion of the bending wires 22 and device wire 23 produced by manipulation of the operating portion 60 can be transmitted reliably to the bending portion 4 as well as to the forceps 30 or an electric scalpel 36.

Also, even if the outside diameter of the sheath and wire unit 5 is minimized, the sheath and wire unit 5 is free of twisting and the operating axis is free of runout. This enables more intuitive operation.

Also, the bending portion 4 is made up of the hinge members 10 engaged convexo-concavely with one another, large sliding surfaces are secured between the projections 44 and recess 42, and the wall thickness of the projections 44 is increased as much as possible, and thus the bending portion 4 can fully withstand any load exerted by the grasping motion of the forceps 30 or the resection motion of the electric scalpel 36.

Also, regarding the bending wires 22 and device wire 23, description has been given of a case in which the bending wire 22 is a stranded wire formed by twisting together nineteen stainless steel strands while a stranded wire formed by twisting together seven stainless steel strands is used as the device wire 23, but each of a bending wire 22a and device wire 23a may be constructed by welding a swaging wire 28 to one end of a solid wire 27 of stainless steel as shown in FIGS. 13(b) and 13(c). As shown in FIG. 13(c), the swaging wire 28 is formed by twisting together nineteen stainless steel strands and then swaging together the twisted strands by the application of pressure on an outer circumference. In this case, note that preferably surface treatment is applied to those portions of the outer circumferences of the solid wire 27 and swaging wire 28 which are inserted into the inner sheaths except for a distal side of the swaging wire 28 and a proximal side of the solid wire 27.

This configuration enables an arrangement in which the swaging wire 28 is passed through a portion corresponding to the bending portion 4 and the solid wire 27 is passed through a portion corresponding to the sheath and wire unit 5. Consequently, the passage of the solid wire 27 makes the sheath and wire unit 5 resistant to elongation and the bending portion 4 is provided with a structure having improved bendability. Note that since the swaging wire 28 has a structure in which the twisted strands are swaged together as described above, in welding the swaging wire 28 to the solid wire 27, it is possible to prevent filler metal from flowing out into space among the wires due to capillary attraction and thereby improve weldability.

Whereas a preferred embodiment of the present invention has been described above, the technical scope of the present invention is not limited to the description of the above embodiment. Various changes or improvements can be made to the above embodiment.

In relation to the bending treatment instrument according to the present embodiment, description has been given of a case in which the forceps-equipped bending treatment instrument 1a and scalpel-equipped bending treatment instrument 1b are used at the same time by being inserted into the endoscope channel 2a and treatment instrument passage tube 2b, respectively, but only one of the forceps-equipped bending treatment instrument 1a and scalpel-equipped bending treatment instrument 1b may be used.
Alternatively, for example, a clip-equipped bending treatment instrument, exclusion bending treatment instrument, or needle-carrier bending treatment instrument may be used other than the forceps-equipped bending treatment instrument 1a and scalpel-equipped bending treatment instrument 1b. Also, in relation to the bending treatment instrument according to the present embodiment described above, description has been given of a case in which the bending-wire passage holes 45 are formed at four locations in the hinge member 10, the number of bending-wire passage holes 45 may be changed as appropriate. Furthermore, in relation to the bending treatment instrument according to the present embodiment described above, description has been given of a case in which the device-wire passage holes 46 are each formed in an elliptical shape, the device-wire passage hole 46 may be formed, for example, into a circular shape as long as a sufficient motion range is secured for the device wire 23.

It will be apparent from the description in the appended claims that any form resulting from such changes or improvements is also included in the technical scope of the present invention.

The invention is defined solely by the following claims.

### Reference Signs List

- 1a: Forceps-equipped bending treatment instrument
- 1b: Scalpel-equipped bending treatment instrument
- 2: Flexible endoscope
- 2a: Endoscope channel
- 2b: Treatment instrument passage tube
- 3: Patient
- 3a: Affected part
- 4: Bending portion
- 5: Sheath and wire unit
- 10: Hinge member
- 11: Hinge top
- 12: Hinge end
- 13: Hinge base
- 14a, 14b: Displacement prevention member
- 15: Claw
- 16: Groove
- 20: Sheath
- 21: Inner sheath
- 22: Bending wire
- 23: Device wire
- 24: Outer sheath
- 25: Liner blade
- 26: Protective tube
- 30: Forceps
- 31: Forceps blade
- 32: Mobile body
- 33: Pin
- 34: Open/close wire
- 35: Forceps base
- 36: Electric scalpel
- 60: Operating portion
- 61: Grip
- 62: Base
- 63: Operating portion body
- 64: Slider mechanism

## Claims

1. A hinge member (10) substantially tubular in shape, where a plurality of the hinge members (10) make up a bending treatment instrument (1) by being arranged in a row substantially coaxially with one another, the hinge member (10) comprising:
a recess (42) formed along a radial direction of the hinge member (10) at least on an outer edge of an end face (41) on a proximal side in an axial direction; and a pair of projections(44) formed along the radial direction of the hinge member (10), at least on outer edges of an end face (43) on a distal side by protruding therefrom and by being shifted 90 degrees in a circumferential direction from the recess (42), wherein each of the projections (44) protrudes by forming a substantially arc shape and the recess (42) is formed into an arc shape corresponding to the projections (44) so as to be able to come into sliding contact with the projections (44); wherein
a bending-wire passage hole (45) penetrating the hinge member (10) in the axial direction is formed at a position at the projections (44) avoiding sliding surfaces of the projections (44) on the end face (43) on the distal side with respect to the recess (42);
the hinge member (10) further comprising a device-wire passage hole (46) penetrating an axial center portion; **characterised in that**
the bending-wire passage hole (45) is formed at opposite ends of the pair of the projections (44) for a total of four; and
the hinge member (10) has a first bending axis extending along an opposing direction of the pair of projections (44) and passing through substantially center of the arc shape, and a second bending axis extending along an extending direction of the recess (42) and passing through substantially center of the arc shape, wherein the bending-wire passage holes (45) do not intersect with either of the first bending axis and the second bending axis.

2. The hinge member (10) according to claim 1, wherein the device-wire passage hole (46) is formed into a substantially elliptical shape in a cross section orthogonal to the axial direction.

3. The hinge member (10) according to claim 2, wherein the substantially elliptical shape of the device-wire passage hole (46) is formed such that a major axis is parallel to a direction orthogonal to an opposing direction of the projections.

4. The hinge member (10) according to claim 1, wherein the bending-wire passage holes (45) increase in diameter toward the end faces (43) on the distal side and the proximal side from a center portion of the bending-wire passage hole (45) along the axial direction.

5. The hinge member (10) according to claim 4, wherein the recess (42) includes a curved portion (42a) and a straight portion (42b) extending from an end portion of the curved portion (42a).

6. The hinge member (10) according to claim 5, wherein a curvature of the curved portion (42a) is substantially equal to a curvature of a tip of each of the projections (44).

7. The hinge member (10) according to claim 1, wherein notches (44b) facing each other are formed, respectively, at those positions on both flanks of each of the projections (44) which are continuous with the end face (43) on the distal side.

8. The hinge member (10) according to any one of claims 4 to 7, wherein:
the recess (42) and the projections (44) are each formed into an arc shape; and
a curvature of the recess (42) is larger than a curvature of the projections (44).

9. The hinge member (10) according to any one of claims 1 to 8, wherein a diameter size in the cross section orthogonal to the axial direction of the hinge member (10) is 3.8 mm or below.

10. A bending treatment instrument (1) comprising a bending portion (4) formed by assembling the hinge members (10) according to any one of claims 1 to 9.

11. The bending treatment instrument (1) according to claim 10, wherein the bending treatment instrument (1) is inserted into an endoscope channel.

## Patentansprüche

1. Ein Gelenkelement (10), welches im Wesentlichen rohrförmig ist, wobei eine Mehrzahl der Gelenkelemente (10) ein Biegebehandlungsinstrument (1) bilden, indem sie in einer Reihe im Wesentlichen koaxial miteinander angeordnet sind, wobei das Gelenkelement (10) aufweist:
eine Aussparung (42), welche entlang einer Radialrichtung des Gelenkelements (10) zumindest an einem äußeren Rand einer Endfläche (41) auf einer proximalen Seite in einer Axialrichtung ausgebildet ist; und ein Paar von Vorsprüngen (44), welche entlang der Radialrichtung des Gelenkelements (10) zumindest an äußeren Rändern einer Endfläche (43) auf einer distalen Seite ausgebildet sind, indem sie davon vorstehen und in einer Umfangsrichtung von der Aussparung (42) aus um 90 Grad verschoben sind, wobei jeder der Vorsprünge (44) durch Ausbilden einer im Wesentlichen Bogenform vorsteht und die Aussparung (42) in einer Bogenform ausgebildet ist, welche so mit den Vorsprüngen (44) korrespondiert, dass sie dazu imstande ist, in Gleitkontakt mit den Vorsprüngen (44) zu kommen, wobei
ein Biegedraht-Durchgangsloch (45), welches das Gelenkelement (10) in der Axialrichtung durchdringt, an einer Position an den Vorsprüngen (44) unter Vermeidung von Gleitflächen der Vorsprünge (44) an der Endfläche (43) auf der distalen Seite bezüglich der Aussparung (42) ausgebildet ist
wobei das Gelenkelement (10) ferner ein Gerätedraht-Durchgangsloch (46), welches einen Axialmittelabschnitt durchdringt, aufweist, **gekennzeichnet dadurch, dass**
das Biegedraht-Durchgangsloch (45) an gegenüberliegenden Enden des Paars der Vorsprünge (44) insgesamt viermal ausgebildet ist, und
das Gelenkelement (10) eine erste Biegeachse, welche sich entlang einer Gegenrichtung des Paars der Vorsprünge (44) erstreckt und durch im Wesentlichen das Zentrum der Bogenform verläuft, und eine zweite Biegeachse, welche sich entlang einer Erstreckungsrichtung der Aussparung (42) erstreckt und durch im Wesentlichen das Zentrum der Bogenform verläuft, aufweist, wobei die Biegedraht-Durchgangslöcher (45) keine von der ersten Biegeachse und der zweiten Biegeachse kreuzen.

2. Das Gelenkelement (10) gemäß Anspruch 1, wobei das Gerätedraht-Durchgangsloch (46) in einer im Wesentlichen elliptischen Gestalt in einem zur Axialrichtung senkrechten Querschnitt ausgebildet ist.

3. Das Gelenkelement (10) gemäß Anspruch 2, wobei die im Wesentlichen elliptische Gestalt des Gerätedraht-Durchgangslochs (46) so ausgebildet ist, dass eine Hauptachse parallel zu einer zu einer Gegenrichtung der Vorsprünge senkrechten Richtung ist.

4. Das Gelenkelement (10) gemäß Anspruch 1, wobei die Biegedraht-Durchgangslöcher (45) sich von einem Mittelabschnitt des Biegedraht-Durchgangslochs (45) aus entlang der Axialrichtung hin zu den Endflächen (43) auf der distalen Seiten und der proximalen Seite im Durchmesser vergrößern.

5. Das Gelenkelement (10) gemäß Anspruch 4, wobei die Aussparung (42) einen gekrümmten Abschnitt (42a) und einen geraden Abschnitt (42b), welcher sich von einem Endabschnitt des gekrümmten Abschnitts (42a) aus erstreckt, aufweist.

6. Das Gelenkelement (10) gemäß Anspruch 5, wobei eine Krümmung des gekrümmten Abschnitts (42a) im Wesentlichen gleich einer Krümmung einer Spitze von jedem der Vorsprünge (44) ist.

7. Das Gelenkelement (10) gemäß Anspruch 1, wobei einander gegenüberliegende Einkerbungen (44b) jeweilig an jenen Positionen an beiden Flanken von jedem der Vorsprünge (44), welche mit der Endfläche (43) auf der distalen Seite zusammenhängen sind, ausgebildet sind.

8. Das Gelenkelement (10) gemäß irgendeinem der Ansprüche 4 bis 7, wobei:
die Aussparung (42) und die Vorsprünge (44) jeweilig in einer Bogengestalt ausgebildet sind, und
eine Krümmung der Aussparung (42) größer als eine Krümmung der Vorsprünge (44) ist.

9. Das Gelenkelement (10) gemäß irgendeinem der Ansprüche 1 bis 8, wobei eine Durchmessergröße im Querschnitt senkrecht zur Axialrichtung des Gelenkelements (10) 3,8 mm oder darunter beträgt.

10. Ein Biegebehandlungsinstrument (1), aufweisend einen Biegeabschnitt (4), welcher durch Zusammensetzen der Gelenkelemente (10) gemäß irgendeinem der Ansprüche 1 bis 9 ausgebildet ist.

11. Das Biegebehandlungsinstrument (1) nach Anspruch 10, wobei das Biegebehandlungsinstrument (1) in einen Endoskopkanal eingebracht ist.

## Revendications

1. Élément d'articulation (10) de forme sensiblement tubulaire , dans lequel une pluralité d'éléments d'articulation (10) compose un instrument de traitement de pliage (1), en étant agencés dans une rangée de manière sensiblement coaxiale les uns par rapport aux autres, l'élément d'articulation (10) comprenant :
un évidement (42) formé le long d'une direction radiale de l'élément d'articulation (10) au moins sur un bord externe d'une face d'extrémité (41) sur un côté proximal dans une direction axiale ; et une paire de saillies (44) formée le long de la direction radiale de l'élément d'articulation (10), au moins sur les bords externes d'une face d'extrémité (43) sur un côté distal en faisant saillie de ce dernier et en étant décalée de 90 degrés dans une direction circonférentielle à partir de l'évidement (42), dans lequel chacune des saillies (44) fait saillie en formant sensiblement une forme d'arc et l'évidement (42) est formé en forme d'arc correspondant aux saillies (44) afin de pouvoir venir en contact coulissant avec les saillies (44) ; dans lequel :
un trou de passage de fil de pliage (45) pénétrant dans l'élément d'articulation (10) dans la direction axiale est formé dans une position au niveau des saillies (44) en évitant les surfaces coulissantes des saillies (44) sur la face d'extrémité (43) du côté distal par rapport à l'évidement (42) ;
l'élément d'articulation (10) comprenant en outre un trou de passage de fil de dispositif (46) pénétrant dans une partie centrale axiale ; **caractérisé en ce que** :
le trou de passage de fil de pliage (45) est formé au niveau des extrémités opposées de la paire de saillies (44) pour un total de quatre ; et
l'élément d'articulation (10) a :
un premier axe de pliage s'étendant le long d'une direction opposée de la paire de saillies (44) et passant sensiblement par le centre de la forme d'arc, et un second axe de pliage s'étendant le long d'une direction d'extension de l'évidement (42) et passant sensiblement par le centre de la forme d'arc, dans lequel les trous de passage de fil de pliage (45) ne coupent pas le premier axe de pliage ni le second axe de pliage.

2. Élément d'articulation (10) selon la revendication 1, dans lequel le trou de passage de fil de dispositif (46) est formé en une forme sensiblement elliptique dans une section transversale orthogonale à la direction axiale.

3. Élément d'articulation (10) selon la revendication 2,
dans lequel la forme sensiblement elliptique du trou de passage de fil de dispositif (46) est formée de sorte qu'un axe majeur est parallèle à une direction orthogonale à une direction opposée aux saillies.

4. Élément d'articulation (10) selon la revendication 1, dans lequel le diamètre des trous de passage de fil de pliage (45) augmente du point de vue du diamètre vers les faces d'extrémité (43) sur le côté distal et le côté proximal à partir d'une partie centrale du trou de passage de fil de pliage (45) le long de la direction axiale.

5. Élément d'articulation (10) selon la revendication 4, dans lequel l'évidement (42) comprend une partie incurvée (42a) et une partie droite (42b) s'étendant à partir d'une partie d'extrémité de la partie incurvée (42a).

6. Élément d'articulation (10) selon la revendication 5, dans lequel une courbure de la partie incurvée (42a) est sensiblement égale à une courbure d'une pointe de chacune des saillies (44).

7. Élément d'articulation (10) selon la revendication 1, dans lequel des encoches (44b) se faisant face sont formées, respectivement, dans ces positions sur les deux flancs de chacune des saillies (44) qui sont continus avec la face d'extrémité (43) sur le côté distal.

8. Élément d'articulation (10) selon l'une quelconque des revendications 4 à 7, dans lequel :
l'évidement (42) et les saillies (44) sont chacun formés en une forme d'arc ; et
une courbure de l'évidement (42) est supérieure à une courbure des saillies (44).

9. Élément d'articulation (10) selon l'une quelconque des revendications 1 à 8, dans lequel une taille diamétrale dans la section transversale orthogonale à la direction axiale de l'élément d'articulation (10) est de 3,8 mm ou inférieure.

10. Instrument de traitement de pliage (1) comprenant une partie de pliage (4) formée en assemblant les éléments d'articulation (10) selon l'une quelconque des revendications 1 à 9.

11. Instrument de traitement de pliage (1) selon la revendication 10, dans lequel l'instrument de traitement de pliage (1) est inséré dans un canal d'endoscope.
